# EUROPEAN PATENT APPLICATION

(11) **EP 3 542 781 A1**
(43) Date of publication of application: **25.09.2019**
(21) Application number: 17871960.5
(22) Date of filing: 09.11.2017
(51) Int. Cl.: A61K 8/03, A61Q 5/12, A61Q 5/02, A61Q 5/04

(54) **COSMETIC PREPARATION HAVING ENHANCED VISCOSITY FOR TREATING KERATINIC FIBRES, METHOD FOR INCREASING THE VISCOSITY OF A COSMETIC PREPARATION, USE OF VISCOSITY-MODIFYING INGREDIENTS AND METHOD FOR TREATING KERATINIC FIBRES**

(30) Priority: 17.11.2016 US 201615354129
(71) Applicant: Natura Cosméticos S.A., 05106-000 São Paulo - SP (BR)
(72) Inventor: KEIKO ALVES WADA, Joeni, 05106-000 São Paulo - SP (BR); RUIZ MAZIN, Mariana, 05106-000 São Paulo - SP (BR); LIMA GUSMÃO, Edjane, 05106-000 São Paulo - SP (BR); DE ANDRADE FREGONESI, Adriana, 05106-000 São Paulo - SP (BR); ARANDAS MONTEIRO E SILVA, Silas, 05106-000 São Paulo - SP (BR)
(74) Representative: Simmons & Simmons
(86) International application number: PCT/BR2017/050339
(87) International publication number: WO 2018/090116

(57) **Abstract**

The present invention relates to a cosmetic preparation with improved viscosity for treatment of keratin fibers, comprising the mixture of a first cosmetic mask composition and a second potentiating cosmetic composition, which is intended for the treatment of keratin fibers. The present invention further contemplates a process for increasing viscosity of said cosmetic preparation from the use of viscosity modifying ingredients, as well as a method for treating keratin fibers.

## Description

### FIELD OF THE INVENTION

The present invention relates to a cosmetic preparation with improved viscosity for treating keratin fibers comprising the mixture of a first cosmetic mask composition and a second potentiating cosmetic composition, which is intended for treating keratin fibers. The present invention further contemplates a process for increasing the viscosity of said cosmetic preparation from the use of viscosity modifying ingredients, as well as a method for treating keratin fibers.

### BACKGROUND OF THE INVENTION

Daily, hair is subjected to various types of wear induced by physical and environmental factors, such as exposure to UV radiation, heat of the air-dryer, tension when combing, coming, etc. The intensity of the damage increases when the hairs also undergo chemical processes such as decoloring, tinting, smoothing, and permanent treatments, chiefly when the procedures are carried out frequently.

Exposure to these stresses alters the physicochemical properties of the hair, both externally and internally, making them more fragile. In a decoloring process, for instance, there is damage both on the cuticle (outer part) and the cortex (inner part), occurring breakage of di-sulfide bridges (about 15 - 25% in a slight decoloring and about 45% when it is more intense) and impairing the stability of the keratin.

Since these actions damage the cuticle and the cortex of the hair, making the hair brittle, porous, dried out and shininess, it is necessary to provide a cosmetic treatment that will repair the damage of the internal and external portions of the hair, bringing back vitality and beauty to the hair, in a more immediate and intense manner.

Much damaged hair requires a restoring treatment that presents more intense and immediate perceptible result. In general, this type of treatment requires the use of different cosmetic compositions combined, composing a composition kit for intensive treatment.

The knowledge of the viscosity of cosmetic products is an important point in the development of the composition. The measurement thereof enables previous perception of the final performance of the product, both on its stability and the acceptance by the final consumer.

With regard to viscosity, the materials may be classified in Newtonians, when they follow Newton's law for viscous flow (viscosity as ratio between tension and annealing rate), or non-Newtonians, when there is no direct proportion between annealing rate and tension. Crèmes, lotions and emulsions, in general, are included in the second group.

Viscometer is the apparatus used for measuring viscosity. There are various types of viscometers, each with its particularities and applications. Among them are the rotational viscometers, which are much used at present by virtue of their versatility, and they can make readings on products from water to emulsions and gels, depending on the parameters used.

Viscosity modifying agents are widely used in the development of cosmetic compositions, both for achieving better performance of the compositions per se, and for achieving better acceptance by the final consumer.

Knowledge of the performance of the viscosity is particularly important in the case of a composition kit for intensive treatment, the final cosmetic preparation to be applied being prepared by the consumer himself. This is because the perception of increase or decrease in viscosity at the moment of preparation is a critical parameter for acceptance of the product. For example, perception of increase in viscosity in the final cosmetic preparation, with respect to the initial compositions, may be immediately associated to the performance of the product, providing better acceptance thereof.

Therefore, there is the need for technological alternatives for improving the viscosity of cosmetic compositions after they are mixed by the consumer, so as to obtain a final cosmetic preparation for treatment, particularly of keratin fibers.

### BRIEF DESCRIPTION OF THE FIGURES

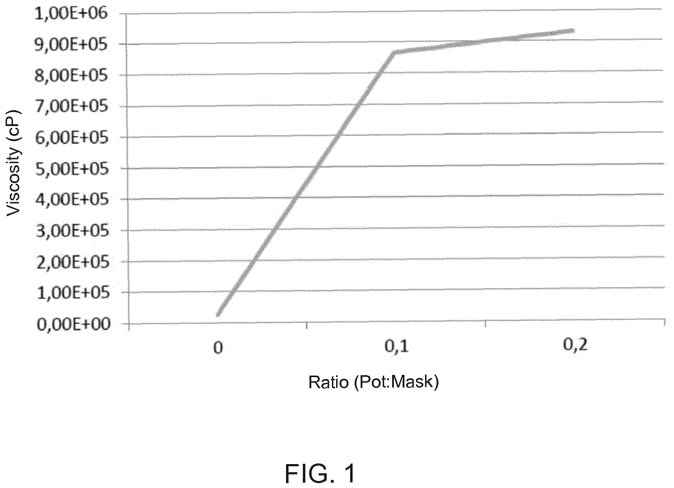

Figure 1 presents the variation of the viscosity as a function of the proportion of potentiating cosmetic composition according to the present invention, added to the cosmetic mask composition also according to the present invention.

### DESCRIPTION OF THE INVENTION

Surprisingly, the applicant found that the use of certain viscosity modifying ingredients, in a specific manner, in treatment cosmetic compositions to be mixed for use by the consumer, provide the increase in viscosity in the final preparation obtained for the treatment of keratin fibers.

Thus, the present invention relates to a cosmetic preparation with improved viscosity, which is intended for the treatment of keratin fibers and formed by mixing two other cosmetic compositions for treatment of keratin fibers.

According to the present invention, "improved viscosity" means greater viscosity than the viscosity of the not yet mixed cosmetic compositions, that is, an increase or gain in viscosity.

"Keratin fibers" means that, in particular and without imposing any limitation, the present invention contemplates human hair.

In this manner, the cosmetic preparation with improved viscosity according to the present invention comprises:
a) a first cosmetic mask composition for treating keratin fibers, comprising a mixture of behetrimonium metosulfate / quaternium 87 / cetearyl alcohol, keratin-fiber conditioning agents, and cosmetically acceptable excipients;
b) a second potentiating cosmetic composition for treatment of keratin fibers comprising a mixture of PEG-150 / decyl alcohol / SMDI copolymer/ propylene glycol / water, keratin fiber conditioning agents and cosmetically acceptable excipients.

"Cosmetic mask composition" means a cosmetic composition in the form of creme, lotion or emulsion that contains conditioning agents capable of conditioning the damaged keratin fiber.

"Potentiating cosmetic composition" means a cosmetic form of viscous liquid, creme, lotion or emulsion that contains conditioning agents capable of potentiating the effect provided by the cosmetic mask composition, densifying the treatment and potentiating the recovery of the hairs, protecting the keratin fiber and prolonging the effects of the chemical processes.

"Cosmetic preparation with improved viscosity" means the mixture of the cosmetic mask composition with the potentiating cosmetic composition that exhibits superior viscosity as compared with the not yet mixed compositions, which provides more intense and immediate restoring treatment.

According to the present invention, the behetrimonium metosulphate / quaternium 87 / cetearyl alcohol may be that made available commercially as Crodazoquat MCC-PA-(MN)™ by Croda Inc, and described in the international patent application published as WO03060046. On the other hand, the mixture of PEG-150 / decyl alcohol / SMDI copolymer / propylene glycol / water, may be that made available commercially as Aculyn™ 44 polymer by Rohm and Haas and described in patent US 4,155,892.

The conditioning agents are those known to a person skilled in the art and, particularly, selected from at least one from *Carya illinoensis, Prunus amygdalus,* hydrolyzed wheat protein, *Avena sativa,* cyclopentasiloxane, dimeticone, dimeticonol, isopropyl palmitate, panthenol, lactic acid, copolymer of hydroxypropyl dimeticone / PPG-8 methylaminopropyl/ PEG-40, stearamidopropyl dimethylamine or mixture of alanine/sodium lactate/arginine/ aspartic acid/ glycine/ serine, isoleucine, PCA/ phenylalanine/ proline/histidine.

The cosmetically acceptable excipients are these known from the prior art, for instance, included in the *The International Cosmetic Ingredient Dictionary and Handbook* (INCI). In particular, the cosmetically acceptable excipients are selected from at least one from cetearyl alcohol, cetrimonium chloride, citric acid, di-sodium EDTA, PEG-90M, phenosyethanol, triethanolamine, perfumes or preservatives.

A second embodiment of the present invention relates to a process for increasing viscosity of a cosmetic composition that comprises adding a potentiating cosmetic composition according to the present invention in a cosmetic mask composition at a ratio of 1:10 to 1:5.

In another embodiment, the present invention also contemplates the use of viscosity modifying ingredients selected from a mixture of behetrimonium metosultate / quaternium 87 /cetearyl alcohol and/ or of a mixture of PEG-150/decyl alcohol/ SMDI copolymer/ propylene glycol/ water in the manufacture of a cosmetic preparation with improved viscosity, as described before, for treatment of keratin fibers.

In another embodiment, the present invention relates to a method for treating keratin fibers, which comprises applying to the keratin fibers a cosmetic preparation as described before.

Optionally, the method for treating keratin fibers comprises additionally application of a finalizing composition in the form of serum that comprises conditioning agents and cosmetically acceptable excipients.

According to the present invention "serum" means a cosmetic form of creme, lotion or emulsion that contains conditioning agents capable of conditioning the damaged keratin fiber.

The method according to the present invention provides a shock treatment, particularly a post-chemical one, that is, with immediate and intense conditioning effect, acting on all the layers of the hairs, repairing damage and prolonging the effects of the chemical processes, by combining the cationic complex of the regenerating mask with the restoration potentiating effect that densifies the treatment and potentiates the recovery of the hairs, protecting the hair fiber and prolonging the effects of the chemical processes. Besides, the treatment complemented with serum seals the hair cuticles, protects against moisture and eliminates the frizz of the hair for up to 24 hours.

The examples below, without imposing any imitation, illustrate the compositions according to the present invention, as well as the effects described herein.

### EXAMPLES

### Example 1. Cosmetic compositions

Tables 1 and 2 hereinafter illustrate the cosmetic compositions according to the present invention.

**Table 1 - Cosmetic compositions according to the present invention**

| Ingredient | Composition A (Mask) | Composition B (Potentiatin g) | Composition C, finalizing and restoring one (Serum - version 1) |
|---|---|---|---|
| Water | 75.119 | 57.54 | 78.142 |
| Mixture of behetrimonium metosulfate / quaternium 87/cetearyl alcohol | 4 | 0 | 0 |
| Oils of seed of *Carya illinoensis* | 0.1 | 0 | 0.5 |
| Protein of *Prunus amygdalus* / hydrolyzed wheat protein / protein of *Avena sativa* (Ceralmilk Premium SP) | 0 | 10 | 0.5 |
| Cetearyl alcohol | 6.5 | 0 | 5.5 |
| Cetrimonium chloride | 4 | 0 | 1 |
| Anhydrous citric acid | 0.25 | 0.1 | 0.01 |
| Cyclopentasiloxane | 0 | 0 | 4 |
| Dimeticone | 0 | 0 | 2 |
| Dimeticonol / TEA-dodecylbenzenesulfate / Benzyl alcohol/lodopropinyl butylcarbamate | 3 | 0 | 2 |
| D-disodium EDTA | 0.05 | 0.05 | 0.05 |
| D-panthenol | 0,5 | 0 | 0 |
| Fragrance | 1.2 | 1.5 | 0.6 |
| lodopropinyl butylcarbamate | 0.1 | 0.1 | 0.1 |
| Isopropyl palmitate | 1.5 | 0 | 0 |
| Lactic acid | 0.17 | 0 | 0.088 |
| PEG-150/decyl alcohol/copolymer SMDI / propylene glycol/ water | 0 | 30 | 0 |
| Copolymer of hydroxypropyl dimeticone /PPG-8 methylaminopropyl /PEG-40 / glycerin/ water/ dipropylene glycol | 0 | 0 | 3 |
| PEG-90M | 0.01 | 0 | 0 |
| Phenoxyethanol | 0.8 | 0.7 | 0.7 |
| Alanine/sodium lactate/ arginine/ aspartic acid/glycine/serine/isoleucine/ PCA/ phenylalanine/proline/histidine (Prodew 500) | 0.7 | 0 | 0.8 |
| Stearamidopropyl dimethylamine | 2 | 0 | 1 |
| Trietanolamine 99w | 0.001 | 0.01 | 0.01 |

**Table 2 - Variations in amounts suitable to the cosmetic compositions according to the present invention**

| Ingredient | Composition C (Mask) minimum-maximum | Composition D (Potentiatin g) minimum-maximum | Composition C Finalizing, restoring (Serum - version 2) minimum-maximum |
|---|---|---|---|
| Water | qsp | qsp | qsp |
| Mixture of behetrimonium metosulfate/quaternium 87/ cetearyl alcohol | 0.1-10,0 | 0 | 0 |
| Oil of seed of *Carya illinoensis* | 0.1-10.0 | | 0.1-10.0 |
| Proteing of *Prunus amygdalus* / hydrolyzed wheat protein / protein of *Avena sativa* (Ceralmilk Premium SP) | 0.001-1.0 | 0.001-1.0 | 0.001-1.0 |
| Cetearyl alcohol | 0.001-10.0 | | 0.001-10.0 |
| Cetrimonium chloride | 0 | 1.0-15.0 | 0.001-10.0 |
| Anhydrous citric acid | 3.0-10.0 | 0 | 3.0-10.0 |
| Ciclopentasiloxane | 0 | 0 | 0.001-10.0 |
| dimeticone | 0.001-10.0 | 0 | 0 |
| Dimeticonol / TEA-dodecylbenzenosulfate / benzyl alcohol / lodopropinyl butylcarbamate | 0 | 0 | 0.001-10.0 |
| Di-sodium EDTA | 0.001-10.0 | 0 | 0.001-10.0 |
| D-panthenol | 0.01-0.05 | 0.01-0.05 | 0.01-0.05 |
| Fragrance | 0.1-5.0 | 0.1-5.0 | 0.1-5.0 |
| lodopropinyl butylcarbamate | 0.001-0.1 | 0.001-0.1 | 0.001-0.01 |
| Isopropyl palmitate | 0.001-10.0 | 0 | 0 |
| Lactic acid | 0.01-1.0 | 0 | 0.01-1.0 |
| PEG-150/decyl alcohol/copolymer SMDI / propylene glycor/water | 0 | 5,0.0-50.0 | 0 |
| Copolymer of hydroxypropyl dimeticone /PPG-8 methylaminopropyl /PEG-40 / glycerin/ water/dipropylene glycol | 0 | 0 | 1.0-10.0 |
| PEG-90M | 0.001-0.01 | 0 | 0 |
| Fhenoxiethanol | 0.001-10.0 | 0 | 0.001-10.0 |
| Alanine/sodium lactate/arginine/asparatic acid/ glycine/ serine/ isoleucine/ PCA/ phenylalanine/ proline / histidine (Prodew 500) | 0.01-0.8 | 0.01-0.8 | 0.01-0.8 |
| Stearamidopropyl dimethylamine | 0.1-10.0 | 0 | 0.1-10.0 |
| Triethanolamine 99w | 0.001-1.0 | 0.001-1.0 | 0.001-1.0 |

### Example 2 - Application of the compositions according to the invention

The composition A according to example 1 was applied to the palm of the hand in an amount sufficient to cover the length of the hair. Then, an amount of the composition B according to example 1 was added to the palm of the hand under the composition A, at a ratio of 10:1, respectively. The compositions A and B were mixed until they formed a consistent and uniform texture.

Then, the cosmetic preparation obtained was applied under the hairs, avoiding the root, and after acting for 3 minutes, the hairs were rinsed abundantly.

To finish, one applied the composition C according to example 1, avoiding the root. The composition C was re-applied during the day.

### Example 3 -Test of increase in viscosity

The test had the objective of evaluating the alteration in viscosity upon adding the composition B (potentiating) according to example 1, which contains the viscosity modifying ingredient PEG-150/decyl alcohol/copolimer SMDI / propylene glycol /water(ACULYN™ 44), to the composition A according to example 1 (mask), which contains the viscosity modifying ingredient behetrimonium metosulfate/quaternium 87/cetearyl alcohol (Crodazoquat MCC-PA-(MH)™), at rations of 1:10 and 1:5.

To evaluate the alteration in viscosity at the rate of 1:10 (0.1) one measured the mass of mask sample in glass bulk of 150 ml and then, measured its viscosity on the digital viscometer Brookfield DV-II+Pro with the following parameters: spindle 4, 6.0 RPM, 50s. Then, one added a proportional amount of potentiating agent and homogenized the mixture. The procedure was carried out in triplicate and repeated at the ratio of 1:5 (0.2), adapting the parameters for reading of higher viscosity: spindle 4, 0.5 RPM, 50s. The temperature of the samples was kept during the study (21°C ± 0.7°C).

The masses measured are shown in the table below:

**Table 2 - Measurement of mass of the samples of potentiating agent and mask for the ratio 1:10 (0,1)**

| Sample | Mask (g) | Potentiating agent (g) | Proportion |
|---|---|---|---|
| 01 | 136.96 | 13.56 | 0.10 |
| 02 | 135.03 | 13.51 | 0.10 |
| 03 | 134.74 | 13.14 | 0.10 |

**Table 3 - Mass measurement of the samples of potentiating agent and mask for ratio 1:5 (0,2)**

| Sample | Mask (g) | Potentiating agent (g) | Proportion |
|---|---|---|---|
| 01 | 134.48 | 26.50 | 0.20 |
| 02 | 135.11 | 26.37 | 0.20 |
| 03 | 139.22 | 27.44 | 0.20 |

For both proportions, one measured the viscosity of the mask without adding potentiating agent and then the mixture of the two products, in triplicate. The results were shown in the table:

**Table 4 - Measurement of viscosity of the samples of potentiating agent and mask for the ratio 1:10**

| Viscosity (cP) / Torque (%) | | | | |
|---|---|---|---|---|
| | Mask | | Mask + Potentiating agent | |
| 01 | 26.500 | 26.4 | 877.000 | 73.5 |
| 02 | 20.600 | 20.6 | 882.000 | 73.1 |
| 03 | 27.200 | 27.2 | 837.000 | 69.8 |

**Table 5 - Measurement of viscosity of the samples of potentiating agent and mask for the ratio 1:5**

| Viscosity (cP) / Torque (%) | | | | |
|---|---|---|---|---|
| | Mask | | Mask + Potentiating agent | |
| 01 | 25.100 | 25.1 | 915.000 | 76.3 |
| 02 | 24.600 | 24.6 | 913.000 | 76.1 |
| 03 | 26.000 | 26.0 | 960.000 | 80.0 |

The results show that in both cases the increase in viscosity upon adding the potentiating agent was significant, being greater for the ratio 1:5. The value obtained for mask + potentiating agent 1:10 was about 35 times as high as that obtained for the mask. In the second case, ratio of 1:5, this value was of about 37 times as high.

In order to view better the variation, one calculated the mean of the values in triplicate and constructed a viscosity graph as a function of the proportion of potentiating agent:mask, where one can observe a great variation with the addition of potentiating agent at the ratio of 1:10 and a less significant variation upon increasing the concentration of this material for the ratio 1:5 (figure 1).

On the basis of the results achieved, one can infer that the combination of the potentiating agent with the mask causes a significant increase in viscosity, as compared to the measurement obtained of the mask without addition of potentiating agent.

### Example 4. Test for substantivity on hair fiber

The level of internal substantivity of cosmetic prototypes in human hair fibers can be evaluated by means of fluorescence microscopy analysis.

In this test, the product is applied to hair strands after 24 hours from application of the products, a few hairs are taken from the strands. The hair fibers are included in an acrylic resin. One made sectional cuts of 10µm in thickness, by using a glass razor blade and Ultramicrotomo. The hair fibers are soaked in solution of Rhodamina B (8µg/ml) for 20 minutes, followed by ringing with deionized water for 1 minute and drying in oven at 45°C for 15 minutes. The optical fluorescence microscopy analysis is carried out.one captures 30 fluorescence images for each study group. The images are processed by software, which express the percentage of fluorescence of the samples.

The dye Rodamina B is cationic and reacts with the active sites of sulfonic acid, formed from the cleaving of the S-S bond of cysteine, caused in the hair relaxing process. Then a fluorescent complex is formed on the hair fiber, which is detected when exposed to the fluorescence microscope.

Below, one presents a table with the results found for the internal actuation profile (in cortex) and on surface (cuticle) of the products evaluated.

**Table 6 - Results achieved for the samples tested in the methodology of hair substantivity**

| Treatment | Surface | | Cortical | |
|---|---|---|---|---|
| | % | N° times | % | N° times |
| Mask (N) | 16C | 1.2 | 20A | 1.3 |
| Serum Potentiating agent | 12C | 1.1 | 29AB | 1.4 |
| Mixture 1 (10:1) | 16C | 1.2 | 36BDEF | 1.6 |
| Mixture 2 (10:2) | 19C | 1.2 | 33BDE | 1.5 |

| | | | | |
|---|---|---|---|---|
| * Different letters indicate significantly different values. | | | | |

The results show that:
- One notices hair filling for all the products;
- There was potentiation of the mixtures proposed (mixture 1 and mixture 2);
- There was no statistic difference between the mixtures.

### Example 5 - Restructuring potential

This technique evaluates the levels of damage on the surface of the hair fiber, by means of electronic scanning microscopy, coupled to the analysis of images.

After 24 hours from application of the products, one removes a few hairs from the strands. The hairs are analyzed by electronic scanning microscopy (ESM/MEV). The images generated are treated by image analysis software, which converts fragments and borders evidenced to binary codes, which is then booked.

The results of the treatments are compared statistically, through t-student tests or ANOVA, always considering interval of reliability of 95%.

The table below summarizes the results found referring to the tested products:

**Table 7 - Results achieved for samples tested in the methodology of hair restructuring potential**

| Treatment | % | N° times |
|---|---|---|
| Mask (N) | 15 A | 1.2 |
| Serum Potentiating agent | 17 A | 1.2 |
| Mixture 1 (10:1) | 19 A | 1.2 |
| Mixture 2 (10:2) | 23 A | 1.3 |
| Serum Finalizing (N) | 30 B | 1.4 |

| | | |
|---|---|---|
| *Different letters indicate significantly different values. | | |

The results show that:
- All the formulations exhibit potential of surface protection to the hair fiber - outer are of the hair;
- The formulas of Mask (N), potentiating agent, Mixture 1 (10:1); Mixture 2 (10:2) do not exhibit statistic differences from each other;
- The serum exhibited a superior performance with respect to the other proportions;

The satisfactory results in both tests (Substantivity/ Restructuring potential) enable us to support:
- Hair recovery;
- Both products act on the internal and external part of the hairs, bringing back mass for the region of the cortex of the hair;
- The potentiation of the effect was identified for the combination Mask and Potentiating agent - mixture 1 and 2, without significant difference between them, it being possible to recognize that the potentiating agent optimized the penetration of the mask into the hair fiber, increasing the replacement of the components in the cortex.

### Example 6 - Test for Softness

In the present study, tinted Caucasian hair strands were subjected to the treatments with the products under investigation.

One carried out the measurements of the compressibility of the strands before and after the treatment, through the Ring Method. For the measurement of compressibility one employed the equipment EMIC DL-500, together with the supports containing the rings 30 and 23 mm in diameter.

The table below summarizes the results found referring to the products tested.

**Table 8 - Results achieved for the samples tested in the hair softness methodology.**

| Treatment | % | N° times |
|---|---|---|
| Mixture 1 (10:1) | 71 | 3.5 |
| Mixture 2 (10:2) | 66 | 2.69 |

The results show that:
- There was no statistically significant difference comparing mixture 1 with mixture 2;
- Hair 3 x softer;
- Hydrated hair;
- Hair 3 x more hydrated.

### Example 6 - Test for Shininess

In the present study, tinted Caucasian hair strands were subjected to the treatments with the products under investigation.

The equipment Glossmeter (BYK Gardner®) was used to obtain the reading of shininess.

One obtained the values of shininess of the dry and clean strands (Initial). After the procedure of applying the products, the strands were dried in a controlled environment at 55 ± 5% U.R. and 22 ± 2 °C for 24 hours and one obtained new measurements of shininess (Final).

The table below summarizes the results found referring to the products tested.

**Table 9 - Results achieved for the samples tested in the hair shininess methodology**

| Treatment | % | N° times |
|---|---|---|
| Mask (N) | 86 | 1.9 |
| Mixture 1 (10:1) | 75 | 1.8 |
| Mixture 2 (10:2) | 81 | 1.8 |

The results show that:
- All the preparations imparted shininess to the hair;
- One did not observe any significance in potentiation of the effect;
- Mixtures 1 and 2 exhibited the same significance of performance.

### Example 7 - Test for Tension and Deformation

In the present study, tinted Caucasian hair strands were subjected to the treatments with the products under investigation.

After application of the products, the strands were dried for 24 hours in a controlled environment at 22 ± 2°C and 55 ± 5% of relative humidity. Then, one evaluated 50 hairs collected at random among the treated strands of each group. Each hair was secured by a lower claw and an upper claw attached to the charge cell of a dynamometer at the upper part. One evaluated the parameter: the parameter: deformation in the rupture and force obtained in the specific deformation of 20%. For this test one used the equipment of EMIC model DL500 provided with a dynamometer with charge cell of 20N.

The table below summarizes the results found referring to the products tested.

**Table 10 - Results achieved for the samples tested in the hair tension and deformation methodology.**

| Treatment | % of Deformation | % of Force |
|---|---|---|
| Mixture 1 (10:1) | 6 | 17 |
| Mixture 2 (10:2) | 5 | 13 |

The results show that:
- There was an increase in the force and deformation of the hair was significant for all the preparations;
- There was no statistic difference between the preparations, including mixture 1 versus mixture 2.

### Example 8 -Anti-frizz Test

In the present study, tinted Caucasian hair strands were subjected to the treatments with the products under investigation.

The strands were photographed right after the treatments and after 24 hours from drying in environment.

The original images, all of them with resolution of 6.0 Mpixels were converted to a gray scale, using the software Scion® Image for Windows (Scion Corp). The Frizz was determined as the percentage in black pixels area referring to the rebellious hairs (detached from the strand body), obtained from the digital image after binarization (for conversion to black/white)

The table below summarizes the results found referring to the products tested.

**Table 11 - Results achieved for the sample tested in the anti-frizz evaluation methodology.**

| Treatment | % Antifrizz | Number of times |
|---|---|---|
| Serum finalizing | 57 | 2.3 times as much |

The results show that:
- Hair with less frizz
- More disciplined hair
- With 50% less frizz
- Less rebellious hairs
- Resistance to moisture

### Example 9. Test for Keeping the Smooth Effect

In the present study, tinted Caucasian hair strands were subjected to the treatments with the products under investigation.

The strands were photographed immediately after smoothing (initial). Then, the strands were subjected to a cycle of successive washings with the products. After 10 consecutive washings, the strands were dried, and the digital images were obtained.

The table below summarizes the results found referring to the products tested.

**Table 12 - Results obtained for the samples tested in the smooth effect keeping methodology**

| Treatment | % | N° times |
|---|---|---|
| Mask (N) | 35 A | 1.5 |
| Serum potentiating | 37 A | 1.6 |
| Mixture 1 (10:1) | 35 A | 1.5 |
| Mixture 2 (10:2) | 38 A | 1.6 |
| Serum finalizing (N) | 44 B | 1.8 |

| | | |
|---|---|---|
| * Different letters indicate significantly different values. | | |

The results show that:
- The products promoted increase in keeping the smooth effect;
- Prolongs the duration of the progressive brushing;
- Helps to keep the progressive brushing;
- Keeps the progressive brushing longer.

### Example 10 - Test for Keeping the Color

In the present study, doubly decolored hair strands were subjected to the coloring procedure, using the coloring mixture: very intense dark reddish blonde, 666 (rubi). Then, the strands were subjected to 30 successive washings with the treatments proposed in the investigation design.

The color measurements were obtained before the tinging (double decolored hair), after the tinging (0 washing) and after 1, 5, 10, 15, 20 and 30 successive washings with the products, using the spectrophotometer Byk-Gardner Spectro-Guide Sphere Gloss.

The table below summarizes the results found referring to the products

**Tale 13 - Results achieved for the samples tested in the color keeping methodology.**

| Treatment | % | N° times |
|---|---|---|
| Mask (N) | 28 A | 1.4 |
| Mixture 1 (10:1) | 27 A | 1.4 |
| Mixture 2 (10:2) | 24 A | 1.3 |
| Serum finalizing (N) | 47 B | 1.9 |

| | | |
|---|---|---|
| *Different letters indicate significantly different values. | | |

The results show that:
- The products exhibited color keeping performance with respect to the control;
- There was no significant difference between the mixtures (1 and 2);
- One understands as synonym sayings: protection of the color; keeping the color of the tinted hair;
- Hair tinted longer; keeping the color.

A persons skilled in the art will know how to evaluate, by means of the teachings contained in the text and in the examples presented, advantages of the invention and propose variations and equivalent alternatives of embodiment, without departing from the scope of the invention, as defined in the accompanying claims.

## Claims

1. A cosmetic preparation with improved viscosity for the treatment of keratin fibers, **characterized by** comprising the mixture of:
a) a first cosmetic mask composition for treatment of keratin fibers, comprising a mixture of behetrimonium metosulfate / quaternium 87 / cetearyl alcohol, keratin fibers conditioning agents and cosmetically acceptable excipients;
b) a second potentiating cosmetic composition for treatment of keratin fibers, comprising a mixture of PEG-150 / decyl alcohol / SMDI copolymer / propylene glycol / water, keratin fibers conditioning agents and cosmetically acceptable excipients.

2. The preparation according to claim 1, **characterized in that** the conditioning agents are at least one from *Carya illinoensis,* Prunus amygdalus, hydrolyzed wheat protein, *Avena sativa,* cyclopentasyloxane, dimeticone, dimeticonol, isopropyl palmitate, panthenol, lactic acid, copolymer of hydroxypropyl dimeticone / PPG-8 methilaminopropyl / PEG-40, stearamidopropyl dimethylamine or mixture of alanine / sodium lactate / arginine / aspartic acid / glycine / serine / isoleucine / PCA / phenylamine / proline / histidine.

3. The preparation according to claim 1, **characterized in that** the cosmetically acceptable excipient include at least one from cetearyl alcohol, cetrimonium chloride, citric acid, di-sodium EDTA, PEG-90M, phenoxyethanol, triethanolamine, perfume or a preservative.

4. The preparation according to claim 1, **characterized in that** the keratin fibers are hairs.

5. A process for increasing the viscosity of a cosmetic preparation, **characterized by** comprising adding a potentiating cosmetic composition for treatment of keratin fibers according to any one of claims 1 to 3 in a cosmetic mask composition for treatment of keratin fibers according to any one of claims 1 to 3 at the ratios 1:10 and 1:5.

6. The process according to claim 5, **characterized in that** the keratin fibers are hairs.

7. Use of viscosity modifying ingredients, **characterized by** being in the manufacture of a cosmetic preparation with improved viscosity for treatment of keratin fibers, wherein said cosmetic preparation is constituted by a first cosmetic mask composition for treatment of keratin fibers and a second potentiating cosmetic composition for treatment of keratin fibers and the viscosity modifiers are one or more from a mixture of behetrimonium metosulfate / quaternium 87 / cetearyl alcohol and a mixture of PEG-150 / decyl alcohol / SMDI copolymer / propylene glycol / water.

8. Use according to claim 7, **characterized in that** at least one viscosity modifier will be contained in each cosmetic composition.

9. Use according to claim 7, **characterized in that** the keratin fibers are hairs.

10. A method for treatment of keratin fibers, **characterized by** comprising applying to the keratin fibers a cosmetic composition according to any one of claims 1 to 4.

11. The method according to claim 10, **characterized in that** the keratin fibers ae hairs.

12. The method according to claim 10, **characterized by** consisting additionally in applying a finalizing composition in the form of serum, which comprises conditioning agents and cosmetically acceptable excipients.

13. The method according to claim 12, **characterized in that** the conditioning agents are selected from at least one of *Carya illinoensis,* Prunus amygdalus, hydrolyzed wheat protein, *Avena sativa,* cyclopentasiloxane, dimeticolne, dimeticonol, isopropyl palmitate, panthenol, lactic acid, copolymer of hydroxypropyl dimeticone /PPG-8 methilaminopropyl / PEG-40, stearamidopropyl dimethylamine or a mixture of alalnine / sodium lactate / arginine / aspartic acid / glycine / serine / isoleucine / PCA / phenylamine / proline / histidine.

14. The method according to claim 12, **characterized in that** the cosmetically acceptable excipients include at least one of cetearyl alcohol, cetrimonium chloride, citric acid, di-sodium EDTA, PEG-90M, phenoxyethanol, triethanolamine, perfume or preservataive.
